(19) Europäisches Patentamt
European Patent Office
Office européen des brevets

(11) **EP 3 773 787 B1**

(12) **EUROPÄISCHE PATENTSCHRIFT**

(45) Veröffentlichungstag und Bekanntmachung des Hinweises auf die Patenterteilung:
**21.02.2024 Patentblatt 2024/08**

(21) Anmeldenummer: **19716339.7**

(22) Anmeldetag: **02.04.2019**

(51) Internationale Patentklassifikation (IPC):
**A61M 1/16** *(2006.01)* **B01D 19/00** *(2006.01)*

(52) Gemeinsame Patentklassifikation (CPC):
**A61M 1/1635; A61M 1/1658; B01D 19/0057; B01D 19/0063;** A61M 2206/16

(86) Internationale Anmeldenummer:
**PCT/EP2019/058317**

(87) Internationale Veröffentlichungsnummer:
**WO 2019/193013 (10.10.2019 Gazette 2019/41)**

(54) **DIALYSEGERÄT**

DIALYSIS MACHINE

APPAREIL DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.04.2018 DE 102018107895**

(43) Veröffentlichungstag der Anmeldung:
**17.02.2021 Patentblatt 2021/07**

(60) Teilanmeldung:
**23205978.2 / 4 289 492**

(73) Patentinhaber: **Fresenius Medical Care Deutschland GmbH
61352 Bad Homburg (DE)**

(72) Erfinder:
• **KLÖFFEL, Peter
97720 Nüdlingen (DE)**
• **STÄBLEIN, Tilman
97072 Würzburg (DE)**
• **SYFONIOS, Andreas
97493 Bergreinfeld (DE)**
• **HÜMMER, Dirk
97520 Hirschfeld (DE)**
• **IRRGANG, Tobias
97633 Aubstadt (DE)**
• **GLASER, Benedict
97421 Schweinfurt (DE)**

(74) Vertreter: **Herrmann, Uwe
Lorenz Seidler Gossel
Rechtsanwälte Patentanwälte
Partnerschaft mbB
Widenmayerstraße 23
80538 München (DE)**

(56) Entgegenhaltungen:
EP-A2- 0 367 252    WO-A2-2017/054923
DE-A1- 1 766 588    DE-A1- 4 329 385
US-A- 4 371 382    US-A1- 2012 316 799
US-A1- 2017 182 233

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Dialysegerät, mit einem zumindest eine Bilanzkammer aufweisenden Bilanziersystem zur volumetrisch exakten Zu- und Abfuhr von Dialyselösung zu und von einem bei Betrieb mit dem Bilanziersystem fluidisch verbundenen Dialysator, einem mit dem Bilanziersystem in Verbindung stehenden Wassereingangssystem zur Versorgung mit frischer Dialysierflüssigkeit, wobei das Wassereingangssystem eine Vorrichtung zur Entgasung von Wasser aufweist, die mit einem Luftabscheider des Dialysgerätes verbunden ist, wobei ein erster Teilbereich des Luftabscheiders als Mischkammer dient und über wenigstens eine Konzentratleitung mit wenigstens einer Konzentratquelle sowie über wenigstens eine Dialysatleitung mit dem Bilanziersystem in Fluidverbindung steht bzw. fluidisch verbindbar ist. Aus der Druckschrift US4371382A ist eine Vorrichtung zur Kontaktierung und Trennung nichtmischbarer Fluide bekannt. Zudem offenbart die Druckschrift DE1766588A1 ein Verfahren und eine Vorrichtung zur Dialyse von Blut.

Das Wassereingangssystem kann optional einen Rezirkulationskreislauf zur Entgasung von Wasser aufweisen.

In dem Wassereingangssystem kann sich ein Luftabscheider befinden, wobei ein erster Teilbereich des Luftabscheiders als Mischkammer dient und über wenigstens eine Konzentratleitung mit wenigstens einer Konzentratquelle sowie über wenigstens eine Dialysatleitung mit dem Bilanziersystem in Fluidverbindung steht bzw. fluidisch verbindbar ist.

Ein derartiges Dialysegerät ist aus der WO 2017/054923 A1 bekannt. Das aus dieser Druckschrift bekannte Dialysegerät ist schematisch in Figur 4 wiedergegeben. Das Gerät gemäß Figur 4 weist einen Behälter 20 auf, der über die Zuleitung 22 mit RO-Wasser befüllt wird. Das Bezugszeichen 10 kennzeichnet einen Rezirkulations- und Entgasungskreislauf, in dem eine Lösung bzw. eine Flüssigkeit, insbesondere RO-Wasser, die zur Herstellung der fertigen Dialyselösung dient, mittels der Pumpe P1 zirkuliert. Neben weiteren Komponenten sind ein Druckbegrenzungsventil V2 sowie der Primärluftabscheider 50, der im Folgenden auch einfach als "Luftabscheider" bezeichnet wird, in dem Rezirkulationskreislauf 10 angeordnet. In dem Rezirkulationskreislauf 10 wird das RO-Wasser entgast und beheizt.

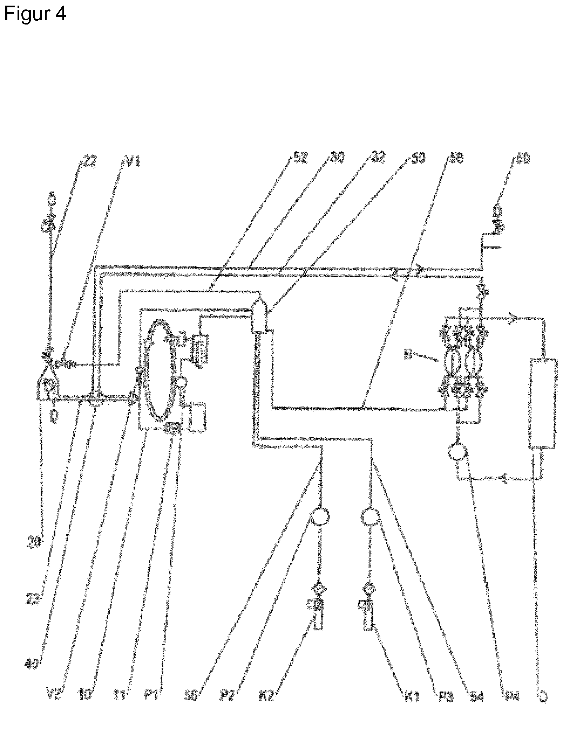

Wie dies aus der Figur 4 weiter hervorgeht, verläuft eine Leitung 23 zwischen dem Behälter 20 und dem Rezirkulationskreislauf 10, über die das in dem Behälter 10 befindliche Fluid, insbesondere RO-Wasser, dem Rezirkulationskreislauf 10 zugeleitet wird.

Von dem Primärluftabscheider 50 zu dem Wassereingangsbehälter 20 verläuft eine Entlüftungsleitung 52, die durch ein Ventil V1 verschließbar ist und bei Bedarf geöffnet wird, um Luft aus dem Luftabscheider 50 zu entfernen. Durch die Abscheidung von Luft soll sichergestellt werden, dass das in dem Rezirkulationskreislauf befindliche Fluid bzw. RO-Wasser möglichst luftfrei ist. Die Luft wird freigesetzt durch die Entgasung des RO-Wassers, indem durch die Entgasungspumpe P1 in Verbindung mit der Entgasungsdrossel 11 ein Unterdruck erzeugt wird, der zum Entgasen des RO-Wassers führt. Eine weitere Quelle für Luft besteht darin, dass einer der Konzentratbehälter K1, K2 leer ist und somit nicht Konzentrat, sondern Luft durch die Konzentratleitungen 54, 56 gefördert wird.

Die Leitungen 30 und 32 kennzeichnen Leitungen für gebrauchtes Dialysat, d.h. Leitungen, die mit dem Dialysator in Verbindung stehen und durch die gebrauchtes Dialysat vom Dialysator strömt. Über die Leitung 32 gelangt das gebrauchte Dialysat zu dem Wärmetauscher 40 und von diesem über die Leitung 30 zu einem Abfluss 60.

Das Bezugszeichen B kennzeichnet das Bilanziersystem, mittels dessen sichergestellt wird, dass das zu dem Dialysator geförderte Dialysat in demselben Volumen zugeführt wird, wie von dem Dialysator gelangendes Dialysat abgeführt wird. Das Bezugszeichen D kennzeichnet den Dialysator, der eine Mehrzahl von Hohlfasermembranen aufweist, die auf der einen Seite von Dialyselösung und auf der anderen Seite von Blut umströmt bzw. durchströmt werden. Wie dies aus der Figur hervorgeht, steht der Dialysator D eingangsseitig und auslassseitig mit dem Bilanziersystem B in Verbindung. Das Bezugszeichen P4 kennzeichnet die Dialysatpumpe, die die Dialyselösung fördert. Diese ist dem hier gezeigten Ausführungsbeispiel stromabwärts des Dialysators D angeordnet. Dadurch wird mittels der Dialysatpumpe P4 das gebrauchte Dialysat in die Bilanzkammer gefüllt. Frisches Dialysat wird über den von der Pumpen P1 erzeugten Flüssigkeitsdruck in die Bilanzkammer gepumpt. Das einstellbare Druckbegrenzungsventil V2 kann zur Einstellung des sogenannten Ladedrucks, der von der Pumpe P1 zum Füllen erzeugt wird, verwendbar sein.

Eine Bilanzkammer kann aus einer Kammer mit vorgegebenen Volumen bestehen, deren zwei Hälften durch eine flexible Membran getrennt werden. Ist zu Beginn eines Bilanzkammerzykluses eine erste Hälfte der Bilanzkammer mit Flüssigkeit gefüllt, so umfasst das Volumen dieser Hälfte das gesamte Bilanzkammervolumen, während sich in der einer zweiten Hälfte weitestgehend oder vollständig keine Flüssigkeit befindet. Wird nun die zweite Hälfte der Bilanzkammer Flüssigkeit gefördert, so verschiebt sich die Membran und die Flüssigkeit wird aus der ersten Hälfte verdrängt. Durch entsprechende Ventilschaltung, die die Zuläufe und Abläufe zu den Bilanzkammerhälften öffnen bzw. verschließen, kann die Richtung des Flusses im bestimmt werden.

Eine Bilanzkammer kann auch anstelle der flexiblen Membran eine starre verschiebbaren Trennplatte aufweisen, die in einem Zylinder verschoben wird. Dabei bilden dann die beiden durch die Trennplatte getrennten Kammern die Bilanzkammerhälften. Solche Bilanzkammern sind auch als Duplex-Pumpen bekannt.

Die Bezugszeichen K1 und K2 kennzeichnen vereinfacht ausgedrückt Konzentratbehälter, bspw. für ein basisches und ein saures Konzentrat. Diese Behälter stehen bei Betrieb des Dialysegeräts über Leitungen 54, 56 mit dem unteren Abschnitt bzw. mit dem Boden des Luftabscheiders 50 in Verbindung. Die Förderung der Konzentrate aus den Konzentratbehältern K1 und K2 erfolgt mittels der Pumpen P2 und P3 durch die Leitungen 54 und 56. Die Verbindung der Leitungen 54 und 56 mit den enstprechenden Konzentratquellen beziehungsweise Konzentratbehältern kann über Konzentratansaugstäbe, die an den Leitungen 54 und 56 angeordnet sind, herstellbar sein. Bei dem Pumpen P2 und P3 handelt es sich vorzugsweise um Membranpumpen.

Die durch die Pumpe P1 bewirkte Rezirkulation in den Rezirkulationskreislauf 10 ist durch den geschlossenen Pfeil in der Mitte des Rezirkulationskreislaufes dargestellt.

Im Betrieb der dargestellten Anordnung wird über die Leitung 23 RO-Wasser und dem unteren Teil des Primärluftabscheiders 50 über die Leitungen 54 und 56 Konzentrate zugeführt. Die Komponenten Wasser und wenigstens ein Konzentrat vorzugsweise zwei oder drei Konzentrate werden zur Mischung in den unteren Teil des Primärluftabscheiders 50 überführt. Diese Komponenten strömen aus dem unteren Bereich des Primärluftabscheiders 50 über die Leitung 58 in das Bilanzkammersystem B. Über die Leitung 23 kann ein ein entsprechend großes Volumen RO-Wassers in den Rezirkulationskreislauf 10 nachströmen.

Der separate Wassereingangskreis, der oben auch als Rezirkulationskreislauf 10 bezeichnet wird, bzw. in einer Ausgestaltung einen solchen Rezirkulationskreislauf aufweist, versorgt die Bilanzkammer B des Gerätes mit temperiertem Wasser. Wird - wie ausgeführt - Lösung aus dem Rezirkulationskreislauf 10 entnommen, d.h. zum Bilanzkammersystem B geführt, wird die entnommene Menge aus der Wassereingangskammer 20 nachgefüllt und über den Wärmetauscher 40 mittels der gebrauchten Dialysierflüssigkeit vorgewärmt.

Wie dies aus Figur 4 weiter hervorgeht, erfolgt die Luftabscheidung aus dem Primärluftabscheider 50 in dessen oberen Bereich, der abweichend von dem unteren Bereich mit dem Rezirkulationskreislauf verbunden ist bzw. dessen Bestandteil bildet. Die Zufuhr von Konzentraten mittels der Leitungen 54, 56 sowie die Abfuhr der fertigen Dialyselösung mittels der Leitung 58 erfolgt aus einem demgegenüber unteren Abschnitt des Primärluftabscheiders.

Diese beiden Abschnitte des Primärluftabscheiders 50 sind mittels einer Trennplatte mit Öffnungen für die Luftabscheidung miteinander verbunden.

Üblicherweise werden die Konzentrate aus den Konzentratbehältern in einzelnen Hüben oder Teilhüben von Membranpumpen in die Mischkammer, d.h. in den ersten Teilbereich des Luftabscheiders gefördert. Das Mischungsverhältnis wird über Anzahl der Hübe bzw. über die Größe der Hübe der Pumpe(n) bestimmt. Die auf diese Weise dem ersten Teilbereich des Luftabscheiders zugeführten Konzentrate vermischen sich jedoch nicht sofort, sondern es liegen Konzentrationsgradienten vor. Wird diese inhomogene Flüssigkeit in eine Bilanzkammerhälfte des Bilanziersystems überführt, besteht die Notwendigkeit, dass das in die Bilanzkammer tatsächlich überführte Volumen an Flüssigkeit tatsächlich das für den anstehenden Hub der Bilanzkammer vorgesehene Konzentrat zumindest weitgehend, am besten vollständig umfasst.

Der vorliegenden Erfindung liegt somit die Aufgabe zugrunde, ein Dialysegerät der eingangs genannten Art dahingehend weiterzubilden, dass die vorgenannte Voraussetzung erfüllt wird, dass also das für den Bilanzkammerhub vorgesehene Konzentrat auch tatsächlich in die mit frischer Dialyselösung zu füllende Bilanzkammerhälfte gelangt.

Diese Aufgabe wird durch ein Dialysegerät mit den Merkmalen des Anspruchs 1 gelöst.

Danach ist vorgesehen, dass das Füllvolumen einer Bilanzkammer der Summe aus dem Volumen der Mischkammer und dem Innenvolumen der Dialysatleitung, die sich von der Mischkammer zu der Bilanzkammer erstreckt, entspricht oder dieses übersteigt. Handelt es sich bei der Leitung von der Mischkammer zur Bilanzkammer um einen oder mehrere Schläuche, gilt:

$$V(MixC) + \text{Innenvolumen des oder der Schläuche} <= V(BC)$$

wobei V(MixC) das Volumen der Mischkammer ist, d.h. das Volumen des ersten Teilbereichs des Luftabscheiders und V(BC) das Füllvolumen einer Bilanzkammer des Bilanziersystems.

Für den Fall, dass V(BC) größer ist als die genannte Summe, kann weiteres Wasser aus dem Rezirkulationskreislauf bzw. aus dem zweiten Teilbereich des Luftabscheiders in die Bilanzkammerhälfte bzw. in die Bilanzkammer gefördert werden.

In jedem Fall kann erfindungsgemäß sichergestellt werden, dass das Mischverhältnis korrekt ist.

Vorzugsweise erfolgt die Taktung des Bilanziersystems und der Konzentratpumpen so, dass letztere aktiviert werden, wenn das Bilanziersystem, d.h. die Bilanzkammer gerade jeweils die leere, mit frischem Dialysat zu versorgende Bilanzkammerhälfte zuschaltet, d.h. zu Beginn eines Bilanzkammerzyklus. Im Hinblick auf den Begriff "leer" wird auf die obigen Ausführungen zur Funktionsweise der Bilanzkammer verwiesen. Sämtliches Konzentrat, das in die Mischkammer, d.h. in den ersten Teilbereich eingeführt bzw. eingespritzt wird, wird direkt in die Bilanzkammerhälfte "mitgerissen", somit erfolgt der Transfer vorzugsweise gleichzeitig.

Vorzugsweise sind eine oder mehrere Pumpen, insbesondere Membranpumpen, Kolbenpumpen etc. zur Förderung von Konzentrat aus der oder den Konzentratquellen in die Mischkammer vorgesehen.

Der Luftabscheider weist vorzugsweise einen zweiten Teilbereich auf, der Bestandteil des Rezirkulationskreislaufes oder des Wassereingangssystems sein kann, wobei von diesem zweiten Teilbereich eine Leitung zur Abfuhr von Luft aus dem Luftabscheider abführt.

Der zweite Teilbereich befindet sich in der Betriebsposition des Luftabscheiders vorzugsweise oberhalb des ersten Teilbereichs, so dass Luft, die sich in dem ersten Teilbereich befindet, durch ein Trennelement hindurch, das den ersten von dem zweiten Teilbereich trennt, in den zweiten Teilbereich gelangt und von dort über die genannte Leitung abgeleitet werden kann. Das Trennelement kann dabei die Funktion erfüllen, dass die zugeführten Konzentrate zumindest weitestgehend im ersten Teilbereich verbleiben.

Wie aus Figur 4 ersichtlich, die teilweise oder vollständig eine Ausführungsform der vorliegenden Erfindung darstellen kann, kann diese Leitung in dem Behälter 20 münden, aus dem der Rezirkulationskreislauf mit Flüssigkeit, insbesondere mit RO-Wasser (RO = Revers Osmose) gespeist wird.

Das Dialysegerät kann weiterhin einen Luftabscheider aufweisen, der einen ersten Teilbereich aufweist, der als Mischkammer dient, wobei der Luftabscheider über Anschlüsse verfügt, mittels derer der erste Teilbereich mit wenigstens einer Konzentratleitung sowie über wenigstens eine Dialysatleitung mit einem Bilanziersystem des Dialysegerätes verbindbar ist, und einen zweiten Teilbereich aufweist, der über wenigstens einen Anschluß verfügt, mittels dessen der zweite Teilbereich mit einem Wasserzulauf des Dialysegerätes verbindbar ist, wobei sich zwischen den Teilbereichen eine zumindest teilweise kegelförmige Trennscheibe befindet, deren kegelförmiger Abschnitt sich in den ersten Teilbereich erstreckt. Der Luftabscheider kann auch über einen oder mehrere Anschlüsse verfügen, mittels derer der zweite Teilbereich mit dem Wassereingangssystem, vorzugsweise mit einem Rezirkulationskreislauf, insbesonders einen Wasserrezirkulationskreislauf des Dialysegerätes verbindbar oder verbunden ist. Der Luftabscheider kann über einer Entgasungsableitung verfügen, mittels dem im Luftabscheider abgeschiedene Luft abführbar ist.

Der Begriff "Trennscheibe" ist allgemein als "Trennelement" in jeder beliebigen Form zu verstehen und nicht auf eine Scheibenform beschränkt, wenngleich nicht ausgeschlossen ist, dass die Trennscheibe bereichsweise scheibenförmig ausgeführt ist.

Die Trennscheibe ist so ausgeführt, dass der kegelförmige Bereich in die Mischkammer, d.h. in den ersten Bereich des Luftabscheiders hineinragt. Unter dem Begriff "kegelförmiger Bereich" ist im Rahmen der Erfindung eine zulaufende, d.h. sich ausgehend von einer Grundfläche in der Querschnittsfläche verkleinernde Struktur zu verstehen, die nicht zwingenderweise kegelförmig ausgeführt sein muss, wenngleich dies eine bevorzugte Ausführungsform der Erfindung ist. Auch sind beispielsweise ein pyramidenförmiger Bereich oder ein Kegel- oder Pydramidenstumpf "kegelförmige Bereiche" im Sinne der Erfindung.

Die Wandungen des kegelförmigen Bereiches können eben bzw. gerade oder gekrümmt sein.

Vorzugsweise weist die Mischkammer eine, zwei oder mehr als zwei Anschlüsse bzw. Zuführungen für das Konzentrat auf. Der kegelförmige Bereich ist vorzugsweise so angeordnet, dass dessen Spitze in Richtung auf die Anschlüsse gerichtet ist.

An den schräg verlaufenden Wänden des kegelförmigen Bereichs kann Luft aufsteigen und z.B. zu Kanälen bzw. Öffnungen geleitet werden, die sich in der Trennscheibe befinden. Vorzugsweise sind diese in einem flachen, flanschartigen Bereich der Trennscheibe angeordnet, der sich um den kegelförmigen Bereich herum erstreckt. Auf diese Weise gelangt die Luft in den zweiten Teilbereich des Luftabscheiders und kann von diesem über eine Leitung oder ein Ventil, etc. abgeschieden werden. Bei einer flachen, d.h. nicht kegelförmigen Struktur besteht die Gefahr, dass sich die Luft unter der Trennscheibe ansammelt. Dem könnte dadurch entgegengewirkt werden, dass eine Vielzahl von Bohrungen in der Trennscheibe vorgesehen wird, was allerdings mit dem Nachteil verbunden wäre, dass unerwünscht viel Konzentrat von dem ersten in den zweiten Teilbereich des Luftabscheiders gelangen würde.

Denkbar ist es, dass während der Rezirkulationsphase durch die erzwungene Konvektion die Durchgangsöffnung oder Durchgangsöffnungen überströmt werden. Luft die sich eventuell im Mischbereich der Kammer befindet, wird durch die Öffnung(en) durch einen Venturi-Effekt aus der Mischkammer gesaugt. Vorzugsweise zeigen die Öffnung(en) in der Trennscheibe in Richtung der Zirkulation im oberen Bereich der Mischkammer, d.h. die Öffnungen liegen schräg in der Trennscheibe.

Die schräge Durchführung der Öffnung(en) in der Trennscheibe führt in der Mischphase, d.h. in der Phase in der die Konzentrate zugespritzt werden, zu einer Zirkulation im Bereich im Mischbereich der Kammer, die das gesamte untere Volumen der Mischkammer erfasst, und für ein fast vollständiges Ausspülen der Mischkammer in der Mischphase sorgt.

Vorzugsweise sind die Öffnungen, d.h. die Durchgangsbohrungen in der Trennscheibe gleichmäßig über deren Kreisradius verteilt (z.B. drei Öffnungen mit einem Abstand von 120°). Diese Anordung sorgt ebenfalls für ein vollständiges Ausspülen des des gesamten unteren Volumens der Mischkammer bzw. der gesamten Mischkammer, wobei Luft, die sich unmittelbar unter der Trennscheibe befindet, in der Rezirkulationsphase durch die eine oder mehreren Öffnungen abgezogen wird.

Vorzugsweise befindet sich in Bezug auf die stehende bzw. auf die Betriebsanordnung des Luftabscheiders der erste Teilbereich unterhalb des zweiten Teilbereichs, was den Vorteil mit sich bringt, dass die zu entfernende Luft selbständig von dem ersten in den zweiten Teilbereich strömt.

Um den Übergang von Luft von dem ersten in den zweiten Teilbereich und umgekehrt die Einströmung von Flüssigkeit bzw. Wasser, vorzugsweise RO-Wasser von dem zweiten in den ersten Teilbereich zu ermöglichen, können sich in der Trennscheibe eine oder mehrere Durchgangsöffnungen befinden, durch die zum Zwecke der Luftabscheidung Luft aus dem ersten Teilbereich in den zweiten Teilbereich und/oder zum Zwecke der Herstellung der Dialyselösung Wasser aus dem zweiten Teilbereich in den ersten Teilbereich gelangt.

Wie oben ausgeführt, ist es bevorzugt, wenn die Trennscheibe einen kegelförmigen Abschnitt und einen sich um diesen herum erstreckenden flanschartigen bzw. scheibenförmigen Abschnitt aufweist, wobei die eine oder mehreren Durchgangsöffnungen in dem flanschartigen bzw. scheibenförmigen Abschnitt angeordnet sind.

Um eine möglichst gute Vermischung bzw. Verwirbelung in der Mischkammer zu erreichen, kann vorgesehen sein, dass der Anschluss des ersten Teilbereichs zur Zufuhr des Konzentrats nicht mit dem Anschluss des ersten Teilbereichs zur Abfuhr des Dialysats fluchtet, sondern z.B. seitlich versetzt dazu angeordnet ist. Aus demselben Grund kann vorgesehen sein, dass der Anschluss des ersten Teilbereichs zur Zufuhr des Konzentrats nicht mit der Kegelspitze fluchtet, sondern versetzt zu dieser angeordnet ist. In diesem Fall trifft das Konzentrat nicht auf die Kegelspitze, sondern auf einen dazu seitlich versetzen Bereich.

Grundsätzlich ist es von Vorteil, dass die Anschlüsse so angeordnet sind, dass in dem ersten Teilbereich ein zirkulierender Flüssigkeitsstrom entsteht.

Der Anschluss bzw. die Anschlüsse, über die das bzw. die Konzentrate in die Mischkammer einleitbar sind, können in die die Mischkammer 110 hineinragen. Vorzugsweise kann seine bzw. ihre Auslasshöhe bzw. Auslasshöhen bei vertikaler Anordnung oberhalb einer Auslassöffnung des Primärluftabscheiders 50 zur Leitung angeordnet sein. Dadurch kann das Risiko, dass evtl. über die Konzentratleitung geförderte Luft, beispielsweise bei leerem Kanister über die Leitung 58 in die Bilanzkammer gelangen kann, reduziert oder eliminiert werden, da die Luft oberhalb vom Auslass 58 zugeführt würde.

Die eine oder die mehreren Durchgangsbohrungen können bezogen auf die stehende Anordnung des Luftabscheiders senkrecht, schräg oder gewunden verlaufen bzw. ausgeführt sein. Denkbar ist es, dass die von dem zweiten Teilbereich eintretende Flüssigkeit mit einem Drall in die Mischkammer eintritt, so dass eine möglichst gute Durchmischung stattfindet.

Vorteilhaft für die Luftabscheidung ist es ferner, wenn zwischen der Spitze des kegelförmigen Abschnitts der Trennscheibe und dem Boden des ersten Teilbereichs, in den der kegelförmige Abschnitt hineinragt, ein Abstand besteht. Vorzugsweise weist der Luftabscheider eine integrative Bauweise auf, indem dieser aus wenigstens zwei Gehäuseteilen zusammengesetzt ist und indem im Bereich der Fügestelle der beiden Gehäuseteile ein abgedichteter Sitz vorgesehen ist, in dem sich die Trennscheibe befindet. Dadurch wird ein Luftabscheider mit einem minimalen Platzbedarf geschaffen. Außerdem wird eine klar definierte und stabile Position der Trennscheibe und somit auch ein definiertes Volumen der Mischkammer erzielt und die Luftabscheideeigenschaften unterliegen nur geringen Schwankungen.

Die Erfindung betrifft des Weiteren ein Verfahren zur Herstellung einer Dialysat mit einem Dialysegerät gemäß einem der Ansprüche 1 bis 11, wobei beim Füllen der Bilanzkammer aus der Mischkammer des Luftabscheiders ein Volumen von Dialysat zugeführt wird, das der Summe aus dem Volumen der Mischkammer des Luftabscheiders und dem Innenvolumen der sich von der Mischkammer zu der Bilanzkammer erstreckenden Dialysatleitung entspricht oder dieses übersteigt. Wie oben ausgeführt, wird so erreicht, dass das gesamte für einen Bilanzkammerhub, d.h. für eine vollständige Füllung einer Bilanzkammer vorgesehene Volumen auch tatsächlich der zu füllenden Bilanzkammer zugeleitet wird und das Mischungsverhältnis der Komponenten untereinander korrekt ist.

Ebenfalls offenbart ist ein Verfahren zur Herstellung einer Dialyselösung mit dem oben beschriebenen Dialysegerät, wobei ein Teil des in der Mischkammer des Luftabscheiders (50) befindlichen Konzentrats dem Wassereingangssystem, wie z.B. dessen Rezirkulationskreislauf - sofern vorhanden - zugeführt wird. Dies lässt sich beispielsweise durch den Abstand der Trennscheibe von der Einführstelle des oder der Konzentrate in die Mischkammer, durch die Größe und/oder Anzahl der Öffnungen in der Trennscheibe, die die beiden Teilbereiche des Luftabscheiders miteinander verbinden, durch die Form des kegelförmigen Bereichs, z.B. durch die Steigung der Wandungen des kegelförmigen Bereichs, durch den Wasserfluss durch das Wassereingangssystem bzw. den Rezirkulationskreislauf, die Konzentrateinspritzgeschwindigkeit, durch die Geschwindigkeit, mit der die Dialyselösung abgeführt wird, etc. erreichen. Der Vorteil des Einströmens von Konzentrat in das Wassereingangssystem bzw. in den Rezirkulationskreislauf besteht darin, dass die Lebensdauer der in dem Wassereingangssystem bzw. Rezirkulationskreislauf befindlichen Bauteile, insbesondere der Entgasungspumpe und dabei insbesondere von deren aus Stahl bestehenden Zahnradpumpenflügeln, gegenüber dem Betrieb in ultrareinem RO-Wasser erhöht wird.

Das Eintreten von Konzentrat in das Wassereingangssystem bzw. in den Rezirkulationskreislauf kann in einem Ausmaß stattfinden, so dass das darin befindliche Wasser bzw. die Lösung eine Leitfähigkeit im Bereich von 0,15 bis 0,2 $\mu$S aufweist.

Die vorliegende Erfindung betrifft des Weiteren ein Dialsegerät nach einem der Ansprüche 1 bis 11, wobei Steuermittel, wie z.B. Pumpen, Ventile etc. vorgesehen sind, die ausgebildet sind die Zufuhrrate an Fluid in die Mischkammer und die Abfuhrrate von Fluid aus der Mischkammer so einzustellen, dass ein Teil der in der Mischkammer befindlichen Flüssigkeit dem Wassereingangssystem zugeleitet wird. Somit verbleibt ein "Nettoüberschuss" an Flüssigkeit, der durch die Trennscheibe bzw. deren Öffnungen in das Wassergangssystem bzw. in den Rezirkulationskreislauf gelangt.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile werden anhand eines in der Zeichnung dargestellten Ausführungsbeispiels näher erläutert.

Es zeigen:

Figur 1: eine Explosionsdarstellung eines Luftabscheiders gemäß der vorliegenden Erfindung,

Figur 2: eine schematische Schnittansicht durch einen Luftabscheider gemäß der vorliegenden Erfindung,

Figur 3: eine perspektivische Ansicht des Luftabscheiders gemäß Figur 1 und 2,

Figur 4: eine schematische Ansicht eines hydraulischen Systems eines Dialysegerätes,

Figur 5: ein Ablaufdiagramm für ein Verfahren zur Herstellung eines Dialysats aus Konzentraten, und

Figur 6: eine schematische Ansicht einer Ausführungsform eines hydraulischen Systems eines Dialysegerätes gemäß der vorliegenden Erfindung.

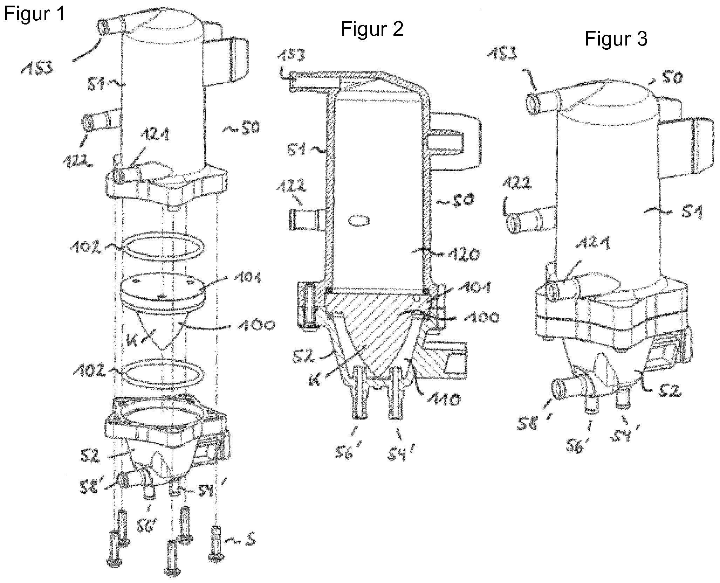

Figur 1 zeigt eine Ausführungsform des Luftabscheiders 50 als Explosionsansicht.

Es wird dadrauf hingewiesen, dass in den Figuren gleiche oder funktionsgleiche Elemente mit denselben Bezugszeichen versehen sind.

Dieser umfasst ein oberes Gehäuseteil 51 und ein mit diesem durch eine Schraubverbindung (vgl. Schrauben S in Figur 1) verbundenes unteres Gehäuseteil 52. Beide Gehäuseteile 51, 52 weisen je einen Flansch auf, wobei die Flansche miteinander verschraubt werden. Zwischen den Gehäuseteilen 51, 52 befindet sich die Trennscheibe 100.

Die Trennscheibe 100 weist eine ebene Oberseite und eine Unterseite auf, von der sich nach unten ein kegelförmiger Bereich K erstreckt. Die Trennscheibe 100 weist darüber hinaus einen scheibenförmigen umlaufenden Flansch 101 auf, der zwischen umlaufenden Dichtungen 102 aufgenommen ist, die oben und unten auf den Flansch 101 gepresst werden, wie dies insbesondere aus Figur 2 hervorgeht.

Unterhalb der Trennscheibe 100 befindet sich der erste Teilbereich 110, d.h. die Mischkammer, und oberhalb der Trennscheibe 100 befindet sich der zweite Teilbereich 120 des Luftabscheiders 50.

Wie dies aus Figur 1 und 2 hervorgeht, weist die Mischkammer zwei Anschlüsse 56' und 54' für die Konzentratleitungen 56 und 54 (vgl. Figur 3) auf. Die beiden Anschlüsse weisen senkrecht nach oben und sind versetzt zur Spitze des kegelförmigen Bereichs K angeordnet. Dieser Versatz sowie die kegelförmige Struktur fördern die Luftabscheidung aus der Mischkammer. Die Luft bewegt sich entlang der Wandung der kegelförmigen Struktur nach oben und gelangt durch Öffnungen in dem Bereich 101 in den zweiten Teilbereich 120.

Senkrecht dazu verläuft der Anschluss 58', an dem die zu dem Bilanziersystem B verlaufende Dialysatleitung 58 angeschlossen wird.

Der Anschluss bzw. die Anschlüsse 56' 54', über die das bzw. die Konzentrate in die Mischkammer 110 einleitbar sind, können in die die Mischkammer 110 hineinragen. Vorzugsweise kann die Auslasshöhe des Anschlusses 56' und/oder des Auslasses 54' bei vertikaler Anordnung oberhalb einer Auslassöffnung 58' des Primärluftabscheiders 50 zur Dialysatleitung 58 angeordnet sein. Dadurch kann das Risiko, dass evtl. über die Konzentratleitung geförderte Luft, beispielsweise bei leerem Kanister über die Leitung 58 in die Bilanzkammer gelangen kann, reduziert oder eliminiert werden, da die Luft oberhalb vom Auslass 58' zugeführt würde.

Der zweite Teilbereich 120 weist einen Zulaufanschluss 121 und einen Ablaufanschluss 122 auf, wobei das RO-Wasser durch den Zulaufanschluss 121 in den zweiten Teilbereich 120 eintritt und nach Durchlaufen des zweiten Teilbereichs 120 durch den Ablaufanschluss 122 wieder aus dem zweiten Teilbereich 120 austritt. Somit stellt der zweite Teilbereich 120 einen Bestandteil des Rezirkulationskreislaufs 10 dar.

Wird RO-Wasser zur Füllung der Bilanzkammerhälfte benötigt, strömt ein Teil des RO-Wassers aus dem Bereich 120 in die Mischkammer 110.

In dem obersten Bereich des zweiten Teilbereichs 120 befindet sich ein Abluftanschluss 123, an dem die Leitung 52 angeschlossen wird, über die die Luft abgeführt wird.

In dem flanschförmigen Bereich 101 der Trennscheibe 100 befinden sich Öffnungen bzw. Durchbrechungen, die die beiden Teilbereiche 110, 120 miteinander verbinden und durch die Luft aus der Mischkammer 110 in den zweiten Teilbereich 120 gelangt und durch die RO-Wasser aus dem zweiten Teilbereich 120 in den ersten Teilbereich 110 gelangt. Es wird dort mit den Konzentraten, vorzugsweise mit einem sauren Konzentrat und mit einem basischen Konzentrat gemischt und dann in eine Bilanzkammerhälfte des Bilanziersystems B geleitet.

Das Bilanziersystem B besteht aus zwei Bilanzkammern, die im Wechsel zueinander betrieben werden, wobei jede der Bilanzkammern aus je zwei Bilanzkammerhälften besteht, die durch eine bewegliche Membran voneinander getrennt sind, so dass bei der Füllung der einen Bilanzkammerhälfte die Entleerung der anderen Bilanzkammerhälfte erfolgt, wie dies aus dem Stand der Technik bekannt ist.

Um sicherzustellen, dass das gesamte von den Membranpumpen 54, 56 in die Mischkammer 110 eingeführte Konzentrat für den betreffenden Bilanzkammerzyklus eingeführt wird, wird bei jedem Bilanzkammerhub ein Flüssigkeitsvolumen in die Bilanzkammer überführt, das größer oder gleich der Summe aus dem Volumen der Mischkammer 110 und dem Volumen der Leitung 58 ist, die sich von der Mischkammer 110 zu der zu befüllenden Bilanzkammer erstreckt.

Eine Zusammenführung von Wasser, dem sauren Konzentrat und dem basischen Konzentrat in der Mischkammer beziehungsweise dann stromab in der Bilanzkammer kann, wie beispielsweise in Figur 4 dargestellt, mittels zweier Konzentrapumpen P2, P3 zur Zuführung des sauren Konzentrats und des basischen Konzentrats umgesetzt werden.

Alternativ dazu können das saure Konzentrat und das basische Konzentrat seuqentiell, d.h. nacheinander, dem Bilanziersystem B zugeführt werden. Dadurch ist es möglich eine Konzentratpumpe einzusparen. Dieser Ansatz ist grundsätzlich aus der WO2018036859 bekannt. Dieser Ansatz der sequentiellen Zudosierung ist dabei nicht auf eine spezielle Hydraulik oder eine spezielle Mischkammer, insbesondere nicht auf die in den Figuren 1 bis 4 dargestellten Ausführungsformen, beschränkt, sondern kann auch unter Nutzung anderer Hydrauliken und Mischkammern eingesetzt werden.

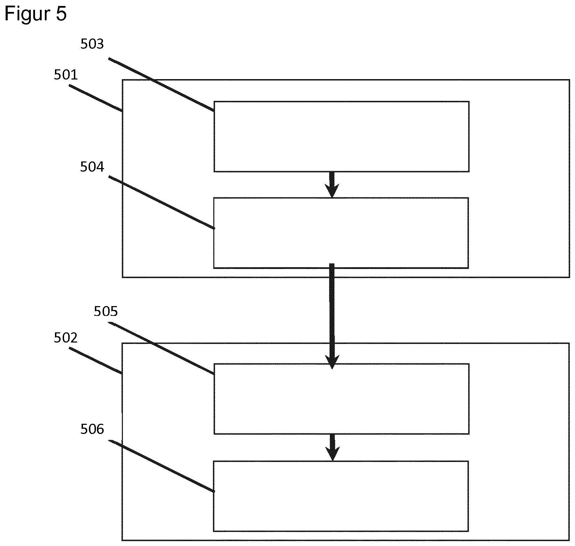

In einer Weiterentwicklung der sequentiellen Zudosierung wurde erkannt, dass folgendes Zudosierungsschema zu einer zuverlässig bestimmten Dialysatzusammensetzung führt. Dabei werden nicht alle Komponenten, saures Konzentrat, basisches Konzentrat und Wasser bereits in der Bilanzkammer zusammengebracht, illustriert anhand Figur 5:

Schritt 1, Referenzzeichen 501 in Figur 5: Das erste Konzentrat wird in einem ersten Schritt mit Wasser in die Bilanzkammer eingebracht, gemischt und aus der Bilanzkammer ausgestossen,
Schritt 2, Referenzzeichen 502 in Figur 5: Das zweite Konzentrat wird mit Wasser in die Bilanzkammer eingebracht, gemischt und aus der Bilanzkammer ausgestossen.

Sowohl Schritt 1 als auch Schritt 2 können jeweils in zwei sequentielle Schritte unterteilt sein. Diese Abfolge hat den Vorteil, dass die sowohl vom ersten Konzentrat als auch vom zweiten Konzentrat durchströmten Leitungen durch das Wasser gespült werden können und so erreichbar ist, dass das gesamte zugeführte Konzentrat in die Bilanzkammer überführt wird und es nicht bereits in der Zuführleitung zur Bilanzkammer zu einer Vermischung zwischen dem ersten Konzentrat und dem zweiten Konzentrat kommt. Bei dem ersten Konzentrat kann es sich um ein saures Konzentrat handeln und bei dem zweiten Konzentrat kann es sich um ein basisches Konzentrat handeln oder umgekehrt:

Schritt 1a, Referenzzeichen 503 in Figur 5: Zuführung des ersten Konzentrats
Schritt 1b, Referenzzeichen 504 in Figur 5: Zuführung des Wassers
Schritt 2a, Referenzzeichen 505 in Figur 5: Zuführung des zweiten Konzentrats
Schritt 2b, Referenzzeichen 506 in Figur 5: Zuführung des Wassers

Bei den sequentiellen Schritten 1a, 1b, 2a, 2b wird immer nur eine Flüssigkeit gefördert, so dass man auch auf die zweite Konzentratpumpe verzichten kann und lediglich mit einer in der Hauptleitung angeordneten Pumpe auskommt, mit der alle Bestandteile, erstes Konzentrat, zweites Konzentrat und Wasser zugeführt werden kann.

Bei einer solchen sequentiellen Zuführung müssen die richtigen Mengen an erstem Konzentrat und zweiten Konzentrat und Wasser zugeführt werden, so dass das fertige Dialysat die richtige Zusammensetzung hat. Dabei ist zu beachten, dass eine Bilanzkammer ein vorgegebenes Volumen hat. Entspricht diese richtige Zusammensetzung beipielsweise für ein Bilanzkammervolumen einem Volumen A des sauren Konzentrats, einem Volumen B des basischen Konzentrats und einem Volumen P des Wasser (mit A+B+P=Bilanzkammervolumen), können bei einer Zuführung gemäß der Schritte 1 und 2 folgende Menge zugeführt werden: Schritt 1: $(2A + P_{rest})$ und Schritt 2: $(2B + P_{rest})$ oder Schritt 1: $(2B + P_{rest})$ und Schritt 2: $(2A + P_{rest})$. Dabei ist $P_{rest}$ jeweils das Volumen an Wasser, das erforderlich ist, um die Bilanzkammer vollständig zu füllen. Dadurch wird erreicht, dass ingesamt das Verhältnis der Komponenten ingesamt zueinander der fertigen Dialysatlösung entspricht, in obigem Beispiel (2 (A+B+P)). Mit anderen Worten - bei dem sequentiellen Zuführen gemäß der Schritte 1 und 2 kann sowohl für das ersten Konzentrat als auch für das zweite Konzentrat die doppelte Menge an Konzentrat zugeführt werden, die erforderliche wäre, um ein Volumen einer Bilanzkammer an fertiger Dialysatlösung herzustellen.

Eine Vermischung der aus der Bilanzkammer ausgestossenen Flüssigkeiten zu einem für die Nutzung fertigen Dialysat kann erreicht werden, indem stromab eine Mischkammer vorgesehen sein. Bei dieser Mischkammer kann es sich um eine separate Kammer nur für diese Funktion vorgesehene kammer handeln. Die Mischkammer kann auch ein Filter sein. Der Filter kann eine semipermeable Membran, beispielsweise in Form einer oder mehrere poröser Platten oder Fasern, aufweisen. Die poröse Membran unterteilt eine Behältnis in eine erste Kammer, in die die Flüssigkeit von der Bilanzkammer kommend zuführbar ist, und eine zweite Kammer, aus der die Flüssigkeit nach Durchtritt durch die Membran ausfliessen kann. Indem die Membran der Flüssigkeit einen Flusswiderstand bietet ist es möglich, dass die nacheinander von der Bilanzkammer zugeführten Flüssigkeiten in der ersten Kammer zumindest teilweise, vorzugsweise vollständig, gemischt werden. Wie anhand Figur 4 ersichtlich, können auch mehrere parallelgeschaltete Bilanzkammern verwendet werden.

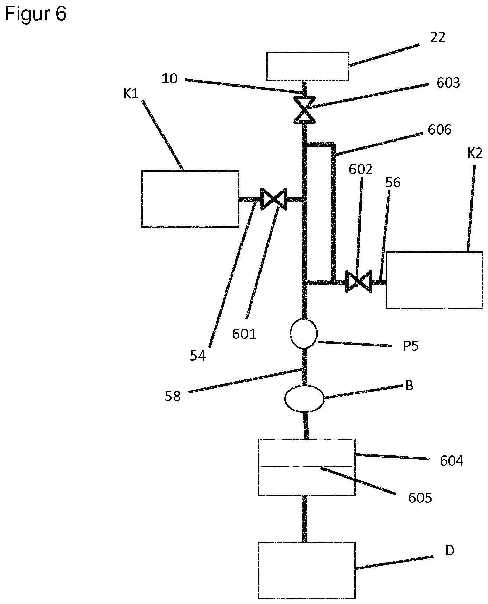

Eine Ausführungsform einer Hydraulik, mit der das serielle Zuführen der Konzentrate und des Wassers umsetzbar ist, ist in Figur 6 illustriert. Elemente, die funktional denen der in Figur 4 gezeigten Hydraulik entsprechen, sind mit denselben Referenzzeichen bezeichnet. Die Hydraulik kann mit eine Steuerung, beispielsweise in Form eines Computers, verbunden sein, die durch entsprechende Ansteuerung der Elemente dir Durchführung der oben beschriebenen Schritte 1 und 2 bzw. 1a, 1b, 2a, 2b steuert.

Die hydraulik weist die Wasserzuleitung 22, verbindbar mit einer Wasserquelle, beispielsweise einer RO Wasserversorgung, und den Rezirkulations- und Entgasungskreislauf 10, das Bilanziersystem B, das zumindest eine Bilanzkammer umfasst, den erste Konzentratbehälter K1 für das erste Konzentrat, den zweiten Konzentratbehälter K2 für das zweite Konzentrat, die erste Konzentratleitung 54 für das erste Konzentrat, die zweite Konzentratleitung 56 für das zweite Konzentrat und die Leitung 58 zum Zuführen der Flüssigkeiten zur Bilanzkammer auf. Dabei ist zu beachten, dass die Leitung 58 gemäß dieser Ausführungsform keine Dialysatleitung im engen Sinne ist, da in dieser Leitung durch die sequentielle Zuführung der Flüssigkeiten, diese nicht alle drei gleichzeitig vorliegen. Während der Behandlung wird die Hydraulik mit dem Dialysator D fluidisch verbunden.

Die sequentielle Zuführung gemäß den oben beschriebenen Schritten 1 und 2 können unter Verwendung separater Pumpen für jede Flüssigkeit, beispielsweise in der Hydraulik der Figur 4 das Wasser mittels der Pumpe P1 und die Konzentrate mittels der Pumpen P2 und P3 durchgeführt werden. Wie oben ausgeführt, erlaubt die sequentielle Zuführung das Einsparen von Pumpen. Wie in Figur 6 illustriert kann eine Pumpe P5 vorgesehen sein. Die Pumpe P5 kann an der Leitung 58 angeordnet sein.

Ventile 601, 602, 603 sind angeordnet an der erste Konzentratleitung 54, der zweite Konzentratleitung 56 und der Wasserzuleitung 10. Über die Steuerung sind diese Ventile ansteuerbar, so dass die Flüssigkeiten sequentiell zugeführt werden. Beispielsweise kann im Schritt 1a, das Ventil 601 geöffnet sein und die Ventile 602 und 603 geschlossen sein. Beim Pumpen mit der Pumpe P5 wird erstes Konzentrat in die Leitung 58 zugeführt. Im Schritt 1b wird das Ventil 601 geschlossen und das Ventil 603 geöffnet. Ventile 602 bleibt geschlossen. Beim Pumpen mit der Pumpe P5 wird Wasser in die Leitung 58 zugeführt und das erste Konzentrat und Wasser der Bilanzkammer B zugeführt. Dadurch, dass im Schritt 1b nur Wasser zugeführt wird, wird aus dem Leitungssystem 58 das erste Konzentrat vollständig entfernt und es befindet sich in dem Leitungsabschnitt 58 nur Wasser. Im Schritt 2a wird das Ventil 603 geschlossen und das Ventil 602 geöffnet. Ventil 601 bleibt geschlossen. Beim Pumpen mit der Pumpe P5 wird zweites Konzentrat in die Leitung 58 zugeführt. Anschließend wird das Gemisch aus Wasser und erstem Konzentrat in Richtung Dialysator aus der Bilankammer B gepumpt. Im Schritt 2b wird das Ventil 602 geschlossen und das Ventil 603 geöffnet. Ventile 601 bleibt geschlossen. Beim Pumpen mit der Pumpe P5 wird Wasser in die Leitung 58 zugeführt und das zweite Konzentrat und Wasser der Bilanzkammer B zugeführt. Anschließend wird das Gemisch aus Wasser und zweiten Konzentrat in Richtung Dialysator aus der Bilankammer B gepumpt. Dadurch, dass im Schritt 2b nur Wasser zugeführt wird, wird aus dem Leitungssystem 58 das erste Konzentrat vollständig entfernt und es befindet sich in dem Leitungsabschnitt 58 nur Wasser.

Die Hydraulik kann, wie oben beschrieben, stromab der Bilanzkammer eine Mischkammer aufweisen. Diese Mischkammer kann ein Filter 604 mit einer semipermeablen Membran 605 sein, die den Filter in zwei Kammern teilt. Von der zweiten Kammer des Filters 604 kann das fertige Dialysat, insbesondere das gemischste Dialysat, dem Dialysator über eine Leitung zuführbar sein.

Stromab der Mischkammer kann eine Leitfähigkeitmesseinrichtung - nicht dargestellt - angeordnet sein. Mittels der Leitfähigkeitsmesseinrichtung kann die richtige Zusammensetzung überwachbar sein, indem beispielsweise die Steuerung die gemessene Leitfähigkeit mit einem Sollwert vergleicht. Eine solche Leitfähigkeitsüberwachung kann erforderlich sein, um bei Betrieb sicherzustellen, dass immer Dialysat der richtigen Zusammensetzung verwendet wird. Um dies zu erreichen wurden bisher immer die richtigen Anteile an Wasser, erstem Konzentrat und zweitem Konzentrat der Bilanzkammer zugeführt. Mit dem hier neu beschriebenen Verfahren und der hier neu beschriebenen Vorrichtung wurde eine Vereinfachung erreicht, indem die Strecke zwischen Bilanzkammer und Dialysator zumindest teilweise, insbesondere die Strecke zwischen Bilanzkammer und Leitfähigkeitssensor zumindest teilweise, zum Mischen des ersten Konzentrats mit dem zweiten Konzentrat genutzt wird.

In einer weiteren Ausführungsform, ebenfalls illustriert in Figur 6 kann die Hydraulik eine Bypassleitung 606 für das Wasser aufweisen. Diese Bypassleitung kann derart angordnet sein, dass Wasser aus der Wasserzuleitung 10 stromab des Ventils 603, mit dem der Wasserzufluss steuerbar ist, in die zweite Konzentratleitung 56 stromab dem Ventil 602, mit dem der Zufluss des zweiten Konzentrats steuerbar ist, zuführbar ist. In dieser Ausführungsform mündet die zweite Konzentratleitung 56 stromab der ersten Konzentratleitung 54 in die Leitung 58, die zur Bilanzkammer führt. Mit anderen Worten, hat die Wasserzuleitung 10 eine Abzweigung zur Bypassleitung 606 und die Bypassleitung 606 hat eine Zulaufstelle in der zweiten Konzentratleitung 56.

Mit dieser Anordnung kann das Wasser im Schritt 2b bei geöffnetem Wasserventil 603 teilweise auch den Anschlußbereich der zweiten Konzentratleitung 56 in die Bilanzkammerzuleitung 58 von dem zweiten Konzentrat freispülen. Damit kann erreicht werden, dass in einem nachfolgenden Schritt 1a, das erste Konzentrat, das entlang der Bilanzkammerzuleitung 58 strömt auch im Anschlußbereich der zweiten Konzentratleitung 56 nicht in Kontakt mit zweitem Konzentrat gelangt. Dies kann erforderlich sein, da es sich bei den beiden Konzentraten um eine konzentrierte Säuren und Basen handeln kann, die miteinander beispielsweise unter Bildung eines Gases oder unter Temperaturerhöhung reagieren können.

**Patentansprüche**

1. Dialysegerät, mit einem zumindest eine Bilanzkammer aufweisenden Bilanziersystem (B) zur volumetrisch exakten Zu- und Abfuhr von Dialyselösung zu und von einem bei Betrieb mit dem Bilanziersystem fluidisch verbundenen Dialysator (D), einem mit dem Bilanziersystem (B) in Verbindung stehenden Wassereingangssystem zur Versorgung mit frischer Dialysierflüssigkeit, wobei das Wassereingangssystem eine Vorrichtung (10) zur Entgasung von Wasser aufweist, die mit einem Luftabscheider (50) des Dialysegerätes verbunden ist, wobei ein erster Teilbereich (110) des Luftabscheiders (50) als Mischkammer dient und über wenigstens eine Konzentratleitung (54, 56) verbindbar mit wenigstens einer Konzentratquelle (K1, K2) sowie über wenigstens eine Dialysatleitung (58) mit dem Bilanziersystem (B) in Fluidverbindung steht, **dadurch gekennzeichnet, dass** das Füllvolumen einer Bilanzkammer der Summe aus dem Volumen der Mischkammer (110) und dem Innenvolumen der Dialysatleitung (58) entspricht oder dieses übersteigt.

2. Dialysegerät nach Anspruch 1, **dadurch gekennzeichnet, dass** eine oder mehrere Pumpen, insbesondere Membranpumpen (P2, P3) zur Förderung von Konzentrat aus der bei Betrieb angeschlossenen Konzentratquelle (K1, K2) in die Mischkammer (110) vorgesehen sind.

3. Dialysegerät nach Anspruch 1 oder 2, **dadurch gekennzeichnet, dass** der Luftabscheider (50) einen zweiten Teilbereich (120) aufweist und das von diesem zweiten Teilbereich eine Leitung (52) zur Abfuhr von Luft aus dem Luftabscheider (50) führt.

4. Dialysegerät nach Anspruch 3, wobei der zweite Teilbereich (120) über einen Zulaufanschluß (121) und eine Entgasungsableitung (153) verfügt, **dadurch gekennzeichnet, dass** sich zwischen den Teilbereichen (110, 120) eine zumindest teilweise kegelförmige Trennscheibe (100) befindet, deren kegelförmiger Abschnitt (K) sich in den ersten Teilbereich (110) erstreckt.

5. Dialysegerät (50) nach Anspruch 4, **dadurch gekennzeichnet, dass** sich in Bezug auf die stehende Anordnung des Luftabscheiders (50) der erste Teilbereich (110) unterhalb des zweiten Teilbereichs (120) befindet, wobei sich bei der stehenden Anordung, die einem Betriebszustand entspricht, die Entgasungsableitung (153) oberhalb der Trennscheibe (100) angeordnet ist und dass sich in der Trennscheibe (100) eine oder mehrere Durchgangsöffnungen befinden, durch die zum Zwecke der Luftabscheidung aus dem ersten Teilbereich (110) in den zweiten Teilbereich (120) und/oder zum Zwecke der Herstellung der Dialyselösung Wasser oder eine Lösung aus dem zweiten Teilbereich (120) in den ersten Teilbereich (110) gelangt.

6. Dialysegerät nach Anspruch 5, **dadurch gekennzeichnet, dass** die Trennscheibe (100) den kegelförmigen Abschnitt (K) und einen sich um diesen herum erstreckenden flanschartigen Abschnitt (101) aufweist und dass die eine oder mehreren Durchgangsöffnungen in dem flanschartigen Abschnitt (101) angeordnet sind.

7. Dialysegerät nach einem der Ansprüche 4 bis 6, **dadurch gekennzeichnet, dass** der Anschluss (54', 56') des ersten Teilbereichs (110) zur Zufur des Konzentrats nicht mit dem Anschluss (58') des ersten Teilbereichs (110) zur Abfuhr des Dialysats fluchtet, sondern versetzt dazu angeordnet ist und/oder dass der Anschluss (54', 56') des ersten Teilbereichs (110) zur Zufuhr des Konzentrats nicht mit der Kegelspitze fluchtet, sondern versetzt zu dieser angeordnet ist.

8. Dialysegerät nach Anspruch 7, **dadurch gekennzeichnet, dass** die Anschlüsse (54', 56', 58') so angeordnet sind, dass in dem ersten Teilbereich (110) ein zirkulierender Flüssigkeitsstrom entsteht.

9. Dialysegerät nach einem der Ansprüche 5 bis 8, **dadurch gekennzeichnet, dass** die eine oder die mehreren Durchgangsbohrungen bezogen auf die stehende Anordnung des Luftabscheiders (50) schräg verlaufen bzw. ausgeführt sind.

10. Dialysegerät nach Anspruch 9, **dadurch gekennzeichnet, dass** die eine oder die mehreren Durchgangsbohrungen derart schräg verlaufen, dass in der Mischkammer (110) eine Zirkulationsströmung ausschließlich oder auch durch die Flüssigkeit gebildet wird, die durch die Durchgangsbohrungen in die Mischkammer (110) eintritt.

11. Dialysegerät nach einem der Ansprüche 4 bis 9, **dadurch gekennzeichnet, dass** zwischen der Spitze des kegelförmigen Abschnitts (K) der Trennscheibe (100) und dem Boden des ersten Teilbereichs (110) ein Abstand besteht.

12. Verfahren zur Herstellung einer Dialyselösung mit einem Dialysegerät gemäß einem der Ansprüche 1 bis 11, **dadurch gekennzeichnet, dass** beim Füllen einer Bilanzkammer aus der Mischkammer des Luftabscheiders (50) ein Volumen von Dialyselösung zugeführt wird, das der Summe aus dem Volumen der Mischkammer (110) des Luftabscheiders (50) und dem Innenvolumen der Dialysatleitung (58) entspricht oder dieses übersteigt.

13. Dialysegerät nach einem der Ansprüche 1 bis 11, **dadurch gekennzeichnet, dass** Steuermittel vorgesehen sind, die ausgebildet sind, die Zufuhrrate an Fluid in die Mischkammer (110) und die Abfuhrrate von Fluid aus der Mischkammer (110) so einzustellen, dass ein Teil der in der Mischkammer (110) befindlichen Flüssigkeit aus der Mischkammer (110) dem Wassereingangssystem zugeleitet wird.

**Claims**

1. A dialysis machine comprising a balancing system (B) that has at least one balancing chamber for a volumetrically exact supply and removal of dialysis solution to and from a dialyzer (D) fluidically connected to the balancing system in operation; a water inlet system connected to the balancing system (B) for the supply of fresh dialysis liquid, with the water inlet system having an apparatus (10) for degassing water that is connected to an air separator (50) of the dialysis machine, with a first part region (110) of the air separator (50) serving as a mixing chamber and being connectable to at least one concentrate source (K1, K2) via at least one concentrate line (54, 56) and being in fluid communication with the balancing system (B) via at least one dialyzate line (58), **characterized in that** the filling volume of a balancing chamber corresponds to or exceeds the sum of the volume of the mixing chamber (110) and the inner volume of the dialyzate line (58).

2. A dialysis machine in accordance with claim 1, **characterized in that** one or more pumps, in particular membrane pumps (P2, P3), are provided for conveying concentrate from the concentrate source (K1, K2) connected in operation into the mixing chamber (110).

3. A dialysis machine in accordance with claim 1 or claim 2, **characterized in that** the air separator (50) has a second part region (120); and **in that** a line (54) for removing air from the air separator (50) leads from this second part region.

4. A dialysis machine in accordance with claim 3, wherein the second part region (120) has an inflow region (121) and a degassing drainage line (153), **characterized in that** an at least partly conical separation disk (100) is located between the part regions (110, 120) whose conical section (K) extends into the first part region (110

5. A dialysis machine in accordance with claim 4, **characterized in that** the first part region (110) is below the second part region (120) with respect to the standing arrangement of the air separator (50), with the degassing drainage line (153) being arranged above the separation disk (100) in the standing arrangement that corresponds to an operating state; and **in that** that one or more passage openings are located in the separation disk (100) through which water or a solution moves from the second part region (120) into the first part region (110) for the purpose of the air separation from the first part region (110) into the second part region (120) and/or for the purpose of the preparation of the dialysis solution.

**6.** A dialysis machine in accordance with claim 5, **characterized in that** that the separation disk (100) has the conical section (K) and a flange-like section (101) extending around it; and **in that** the one or more passage openings are arranged in the flange-like section (101)

**7.** A dialysis machine in accordance with one of the claims 4 to 6, **characterized in that** the connector (54', 56') of the first part region (110) for the supply of the concentrate is not aligned with the connector (58') of the first part region (110) for removing the dialyzate, but is rather arranged offset therefrom; and/or **in that** the connector (54', 56') of the first part region (110) for the supply of the concentrate is not aligned with the cone tip, but is rather arranged offset therefrom.

**8.** An air separator (50) in accordance with claim 7, **characterized in that** the connectors (54', 56', 58') are arranged such that a circulating liquid flow arises in the first part region (110).

**9.** An air separator (50) in accordance with one of the claims 5 to 8, **characterized in that** the one or more passage bores extend or are designed obliquely with respect to the standing arrangement of the air separator (50).

**10.** An air separator (50) in accordance with claim 9, **characterized in that** the one or more passage bores extend obliquely such that a circulation flow in the mixing chamber (110) is only or also formed by the liquid that enters into the mixing chamber (110) through the passage bores.

**11.** An air separator (50) in accordance with one of the claims 4 to 9, **characterized in that** there is a spacing between the tip of the conical section (K) of the separation disk (100) and the base of the first part region (110).

**12.** A method of preparing a dialysis solution using a dialysis machine in accordance with one of the claims 1 to 11, **characterized in that**, on the filling of a balancing chamber from the mixing chamber of the air separator (50), a volume of dialysis solution is supplied that corresponds to or exceeds the sum of the volume of the mixing chamber (110) of the air separator (50) and the inner volume of the dialyzate line (58).

**13.** A dialysis machine in accordance with one of the claims 1 to 11, **characterized in that** control means are provided that are configured to set the supply rate of fluid into the mixing chamber (110) and the removal rate of fluid from the mixing chamber (110) such that some of the liquid located in the mixing chamber (110) is conducted from the mixing chamber (110) to the water inlet system.

**Revendications**

**1.** Appareil de dialyse, comportant un système d'équilibrage (B) présentant au moins une chambre d'équilibre pour l'alimentation et l'évacuation d'un volume exact de solution de dialyse vers et en provenance d'un dialyseur (D) qui, pendant le fonctionnement, est en liaison fluidique avec le système d'équilibrage (B), un système d'entrée d'eau en liaison avec le système d'équilibrage (B) pour l'approvisionnement en liquide de dialyse frais, le système d'entrée d'eau présentant un dispositif (10) destiné au dégazage de l'eau et raccordé à un séparateur d'air (50) de l'appareil de dialyse, une première partie (110) du séparateur d'air (50) servant de chambre de mélange et pouvant être raccordée par le biais d'au moins une conduite de concentré (54, 56) à au moins une source de concentré (K1, K2) et se trouvant en liaison fluidique avec le système d'équilibrage (B) par le biais d'au moins une conduite de dialysat (58), **caractérisé en ce que** le volume de remplissage d'une chambre d'équilibre correspond à la somme du volume de la chambre de mélange (110) et du volume intérieur de la conduite de dialysat (58) ou dépasse celle-ci.

**2.** Appareil de dialyse selon la revendication 1, **caractérisé en ce qu'**une ou plusieurs pompes, en particulier des pompes à membrane (P2, P3), destinées à transporter du concentré depuis la source de concentré (K1, K2) raccordée pendant le fonctionnement jusqu'à la chambre de mélange (110) sont prévues.

**3.** Appareil de dialyse selon la revendication 1 ou 2, **caractérisé en ce que** le séparateur d'air (50) présente une seconde partie (120) et **en ce qu'**une conduite (52) destinée à évacuer de l'air hors du séparateur (50) part de cette seconde partie.

**4.** Appareil de dialyse selon la revendication 3, dans lequel la seconde partie (120) dispose d'un raccord d'admission (121) et d'une évacuation de dégazage (153), **caractérisé en ce qu'**un disque de séparation (100) au moins en partie conique se trouve entre les parties (110, 120), le segment conique (K) dudit disque de séparation s'étendant dans la première partie (110).

5. Appareil de dialyse (50) selon la revendication 4, **caractérisé en ce que**, par rapport à la disposition verticale du séparateur d'air (50), la première partie se trouve sous la seconde partie (120), l'évacuation de dégazage (153) étant disposée au-dessus du disque de séparation (100) dans la disposition verticale, qui correspond à un état de fonctionnement, et **en ce qu'**un ou plusieurs orifices de passage se trouvent dans le disque de séparation (100), par lesquels de l'eau ou une solution passe de la première partie (110) à la seconde partie (120) à des fins de séparation de l'air et/ou de la seconde partie (120) à la première partie (110) à des fins de préparation de la solution de dialyse.

6. Appareil de dialyse selon la revendication 5, **caractérisé en ce que** le disque de séparation (100) présente le segment conique (K) et un segment (101) semblable à une bride s'étendant autour de celui-ci et **en ce que** le ou les plusieurs orifices de passage sont disposés dans le segment (101) semblable à une bride.

7. Appareil de dialyse selon l'une des revendications 4 à 6, **caractérisé en ce que** le raccord (54', 56') de la première partie (110) destiné à alimenter le concentrat n'est pas aligné avec le raccord (58') de la première partie (110) destiné à évacuer le dialysat, mais est disposé décalé par rapport à celui-ci et/ou **en ce que** le raccord (54', 56') de la première partie (110) destiné à alimenter le concentrat n'est pas aligné avec le sommet du cône mais est disposé décalé par rapport à celui-ci.

8. Appareil de dialyse selon la revendication 7, **caractérisé en ce que** les raccords (54', 56', 58') sont disposés de telle sorte qu'un flux de liquide en circulation se forme dans la première partie (110).

9. Appareil de dialyse selon l'une des revendications 5 à 8, **caractérisé en ce que** le ou les plusieurs trous de passage s'étendent ou sont réalisés de manière oblique par rapport à la disposition verticale du séparateur d'air (50).

10. Appareil de dialyse selon la revendication 9, **caractérisé en ce que** le ou les plusieurs trous de passage s'étendent en oblique de telle manière qu'un écoulement de circulation est formé dans la chambre de mélange (110) exclusivement ou aussi par le liquide qui entre dans la chambre de mélange (110) par les trous de passage.

11. Appareil de dialyse selon l'une des revendications 4 à 9, **caractérisé en ce qu'**il y a une distance entre le sommet du segment conique (K) du disque de séparation (100) et le fond de la première partie (110).

12. Procédé de préparation d'une solution de dialyse avec un appareil de dialyse selon l'une des revendications 1 à 11, **caractérisé en ce que**, lors du remplissage d'une chambre d'équilibre, un volume de solution de dialyse est alimenté depuis la chambre de mélange du séparateur d'air (50), lequel volume correspond à la somme du volume de la chambre de mélange (110) du séparateur d'air (50) et du volume intérieur de la conduite de dialysat (58) ou dépasse celui-ci.

13. Appareil de dialyse selon l'une des revendications 1 à 11, **caractérisé en ce que** des moyens de commande sont prévus, lesquels sont conçus pour régler le débit d'alimentation de fluide dans la chambre de mélange (110) et le débit d'évacuation de fluide hors de la chambre de mélange (110) de telle sorte qu'une partie du liquide se trouvant dans la chambre de mélange (110) est amenée dans le système d'entrée d'eau depuis la chambre de mélange (110).

Figur 1

Figur 2

Figur 3

Figur 4

Figur 5

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4371382 A
- DE 1766588 A1
- WO 2017054923 A1
- WO 2018036859 A